# EUROPÄISCHE PATENTANMELDUNG

(11) **EP 1 782 852 A1**
(43) Veröffentlichungstag der Anmeldung: **09.05.2007**
(21) Anmeldenummer: 05024057.1
(22) Anmeldetag: 04.11.2005
(51) Int. Cl.: A61M 5/142

(54) **Anordnung zum automatisierten Zuführen eines flüssigen Medikamentes in den Körper eines Patienten**

(71) Anmelder: F.HOFFMANN-LA ROCHE AG, 4070 Basel (CH); Roche Diagnostics GmbH, 68305 Mannheim (DE)
(72) Erfinder: Bosshard, David, 3251 Wengi (CH); Remde, Axel, 3432 Lützelflüh-Goldbach (CH); Schiltges, Gilbert, 3422 Kirchberg (CH)
(74) Vertreter: Blum, Rudolf Emil

(57) **Zusammenfassung**

Die Erfindung betrifft eine Anordnung zum automatisierten subkutanen Zuführen von Insulin in den Körper eines Patienten. Die Anordnung umfasst eine Infusionskanüle (14) zur subkutanen Einbringung des Insulins in den Körper sowie ein Insulinreservoir (2) mit einer bei kurzfristiger subkutaner Verabreichung für einen Menschen nicht-tödliche Insulinmenge. Des Weiteren umfasst sie eine automatisierte Fördervorrichtung zur Förderung des Insulins vom Reservoir (2) zur Infusionskanüle (14), welche nach manueller Aktivierung durch den Patienten mit einer Verzögerung von vier bis acht Stunden automatisch mit der Förderung des Insulins beginnt und sodann die gesamte im Reservoir (2) bereitgestellte Insulinmenge innerhalb eines Zeitraumes von maximal vier Stunden zur Infusionskanüle (14) fördert.

Durch die Schaffung dieser Anordnung wird eine einfache und komfortable Therapie des Dämmerungsphänomens auch für Diabetespatienten möglich, welche für eine Insulinpumpenanwendung ungeeignet sind oder eine solche nicht wünschen.

## Beschreibung

Die vorliegende Erfindung betrifft ein Verfahren zur automatisierten Zuführung eines flüssigen Medikamentes zu einer Infusionskanüle, eine Anwendung des Verfahrens, eine Anordnung zum automatisierten Zuführen eines flüssigen Medikamentes in den Körper eines Patienten, eine Verwendung der Anordnung, eine Einwegabpackung Insulin als Reservoir für die Anordnung sowie eine Einwegabpackung eines physiologischen Lösungsmittels zur Bildung eines Reservoirs für die Anordnung gemäss den Oberbegriffen der unabhängigen Patentansprüche.

Die automatisierte Zuführung von flüssigen Medikamenten in den Körper kommt bevorzugterweise bei Patienten zur Anwendung, welche einen kontinuierlichen und über den Tag gesehen variierenden Bedarf an einem ausschliesslich subkutan verabreichbaren Medikament haben. Konkrete Anwendungsfälle sind z.B. bestimmte Schmerztherapien und die Behandlung von Diabetes, bei denen am Körper tragbare computergesteuerte Medikamentenpumpen zum Einsatz kommen, welche eine für mehrere Tage ausreichende Medikamentenmenge in einem Reservoir beinhalten und das flüssige Medikament gemäss einem vorprogrammierten Tagesprofil vom Reservoir über eine Infusionskanüle in den Körper des Patienten fördern. Solche Medikamentenpumpen sind jedoch relativ teuer und benötigen, nicht zuletzt aufgrund der Tatsache, dass sie eine potentiell tödliche Medikamentendosis aufnehmen können und mit entsprechenden Sicherheitsfunktionen ausgestattet sein müssen, zu ihrer Anwendung Fachkenntnisse und ein gewisses Geschick im Umgang mit elektronischen Geräten, so dass Ihr Einsatz auf bestimmte Patientengruppen beschränkt ist. Insbesondere bei der Insulintherapie von Diabetes Typ I und Typ II Patienten, welche keine computergesteuerte Insulinpumpe verwenden, sondern das Insulin mehrmals täglich manuell injizieren, ergibt sich das Problem, dass in den frühen Morgenstunden typischerweise ein sehr grosser Insulinbedarf auftritt (Dämmerungsphänomen), also zu einer Zeit, zu welcher der Patient normalerweise schläft. Werden keine speziellen Massnahmen getroffen, so führt dies zu teilweise sehr hohen morgentlichen Blutzuckerwerten und zu einer entsprechend unbefriedigenden Gesamteinstellung mit all ihren bekannten negativen Folgen. Zur Behandlung dieses Dämmerungsphänomens besteht gemäss dem Stand der Technik neben der zuvor erwähnten und nur für eine beschränkte Patientengruppe realisierbaren Insulinpumpentherapie noch die Möglichkeit, abends ein Insulin mit verzögerndem Wirkprofil injizieren, so dass in den Morgenstunden eine genügende Insulinmenge zur Verfügung steht. Dies führt jedoch aufgrund der begrenzten Möglichkeiten zur Realisierung der Wirkprofile häufig nicht zu befriedigenden Ergebnissen. Zudem kann die Wirkung aufgrund von Schwankungen der Resorption sowohl zu früh als auch zu spät einsetzen. Auch besteht noch die Möglichkeit, sich nachts zu einem geeigneten Zeitpunkt (z.B. 4h morgens) wecken zu lassen und eine geeignete Insulinmenge manuell zu injizieren. Dies ist jedoch mit einem erheblichen persönlichen Aufwand und einem entsprechenden Verlust an Lebensqualität verbunden.

Es stellt sich daher die Aufgabe, Verfahren und Vorrichtungen zur Verfügung zu stellen, welche die Nachteile des Standes der Technik nicht aufweisen oder diese zumindest teilweise vermeiden.

Diese Aufgabe wird durch das Verfahren und die Anordnung gemäss den unabhängigen Patentansprüchen gelöst.

Demgemäss betrifft ein erster Aspekt der Erfindung ein Verfahren zur automatisierten Zuführung eines flüssigen Medikamentes zu einer Infusionskanüle, welche bevorzugterweise als Infusionsnadel ausgebildet ist. Dabei wird in einem ersten Schritt das zu verabreichende Medikament in einer bei kurzfristiger (innerhalb von einigen Sekunden) subkutaner Verabreichnung, für einen Menschen nicht-tödlichen Menge in einem Reservoir bereitgestellt. Sodann wird eine automatisierte Fördervorrichtung bevorzugterweise manuell aktiviert, welche mit einer Verzögerung von vier bis acht Stunden damit beginnt, die gesamte bereitgestellte Medikamentenmenge innerhalb von höchstens vier Stunden von dem Reservoir zu der Infusionskanüle zu fördern.

Ein zweiter Aspekt der Erfindung betrifft eine Anordnung zum automatisierten Zuführen eines flüssigen Medikamentes in den Körper eines Patienten, welche bevorzugterweise zur Durchführung des Verfahrens gemäss dem ersten Aspekt der Erfindung geeignet ist. Die Anordnung umfasst eine Infusionskanüle zur subkutanen Einbringung des Medikamentes in den Körper des Patienten, welche bevorzugterweise als Infusionsnadel ausgebildet ist. Zudem umfasst sie ein Reservoir, welches eine bei kurzfristiger (innerhalb einiger Sekunden) subkutaner Verabreichung für einen Menschen nicht-tödliche Menge des zuzuführenden flüssigen Medikamentes und/oder eines physiologischen Lösungsmittels für ein flüssiges Medikament enthält. Des Weiteren umfasst die Anordnung eine automatisierte Fördervorrichtung zur Förderung des flüssigen Medikamentes bzw. des physiologischen Lösungsmittels von dem Reservoir zur Infusionskanüle, welche derartig ausgebildet ist, dass sie nach einer bevorzugterweise manuellen Aktivierung mit einer Verzögerung von vier bis acht Stunden automatisch mit der Förderung beginnt und nach Beginn der Förderung die gesamte im Reservoir bereitgestellte Fluidmenge innerhalb eines Zeitraumes von maximal vier Stunden zur Infusionskanüle fördert.

Die Erfindung weist gegenüber dem Stand der Technik den Vorteil auf, dass das Reservoir keine potentiell tödliche Wirkstoffmenge enthält und bei jeder Anwendung praktisch vollständig entleert wird, so dass auf umfangreiche und komplizierte Sicherheitsmassnahmen verzichtet werden kann. Hierdurch werden einfach zu bedienende und kostengünstige automatische Medikamentenzuführanordnungen möglich, mit denen in Selbstanwendung durch den Patienten zu einem zuvor festgelegten Zeitpunkt ein vorgegebenes Volumen eines flüssigen Medikaments automatisch subkutan appliziert werden kann.

So kann beispielsweise zur Behandlung des Dämmerungsphänomens bei Diabetikern mit der erfindungsgemässen Anordnung die in den Morgenstunden benötigte Insulinmenge zeitrichtig zur Verfügung gestellt werden, ohne dass der Diabetiker die Injektion selbst vornehmen muss. Beim Zubettgehen legt der Patient die Anordnung an, z.B. mittels eines Pflasters oder Befestigungsgurtes, und verbindet deren Kanüle subkutan mit dem Körper, z.B. indem er die als Infusionsnadel ausgebildete Kanüle in den Körper einsticht. Kommt eine Anordnung zur Anwendung, welche im unbenutzten Zustand ein lediglich mit einem physiologischen Lösungsmittel gefülltes Reservoir aufweist, so wird dessen Inhalt bevorzugterweise vor dem Anlegen der Anordnung mit der erforderlichen Menge flüssigen Medikaments, im vorliegenden Beispiel Insulin, ergänzt, was z.B. durch Injizieren des Insulins über ein Septum in das Reservoir erfolgen kann. Zu einem vorgegebenen Zeitpunkt bzw. nach einer vorgegebenen Verzögerungszeit appliziert die Anordnung dann selbstätig die vorgegebene Medikamentenmenge, ohne dass der Diabetiker aufwacht. Als Variante ist es auch vorgesehen, die erfindungsgemässe Anordnung derartig auszugestalten, dass diese die Infusionsnadel erst kurz vor Beginn der Medikamentenförderung in den Körper einsticht, so dass der Patient, welcher zu diesem Zeitpunkt schläft, den Einstich und den hiermit verbundenen Schmerz nicht bewusst wahrnimmt. Nach dem Aufstehen am Morgen legt der Diabetiker die Anordnung ab und führt während des Tages seine Therapie wie gewohnt durch. Bevorzugte Ausführungsformen der erfindungsgemässen Anordnung weisen ein minimales Totvolumen des Überleitsystems zwischen Reservoir und Kanüle auf und werden deshalb bevorzugterweise unmittelbar an der Injektionsstelle am Körper getragen.

Bei einer bevorzugten Ausführungsform des erfindungsgemässen Verfahrens und der erfindungsgemässen Anordnung (im Folgenden summarisch als "die Erfindung" bezeichnet) kommt als anspruchsgemässes Reservoir eine Einwegabpackung eines flüssigen Medikaments oder ein das flüssige Medikament enthaltendes wiederbefüllbares Behältnis zum Einsatz. Im erstgenannten Fall ergibt sich der Vorteil, dass ein Maximum an Sicherheit und Hygiene sichergestellt werden kann, da keine Befüllung des Reservoirs durch den Benutzer erfolgt, im zweiten Fall ergibt sich der Vorteil, dass eine geringere Abfallmenge anfällt und Kosten eingespart werden können. Auch besteht hier die Möglichkeit, die Medikamentenart und -menge exakt an den Medikamentenbedarf des Patienten anzupassen.

Bei einer weiteren bevorzugten Ausführungsform der Erfindung kommt als Reservoir eine Einwegabpakkung eines physiologischen Lösungsmittels zum Einsatz, in welche direkt vor der Verwendung die gewünschte Menge flüssigen Medikamentes eingebracht werden kann. Dazu weisst die Einwegabpackung bevorzugterweise ein Septum auf, welches zwecks Einspritzen des Medikamentes mit der Nadel einer mit dem Medikament gefüllten Spritze durchstochen werden kann. Bevorzugterweise enthält die Einwegabpackung eine physiologische Kochsalzlösung. Bei dieser Ausführungsform ergibt sich der Vorteil, dass die Medikamentenart und -menge individuell angepasst werden kann, bei einem gleichzeitig hohen Mass an Hygiene und Sicherheit, und dass aufgrund des grossen spezifischen Volumens der zu fördernden Medikamentendosis eine gleichmässige bzw. langsame Ausschüttung ohne aufwendige Fördertechnik möglich wird.

Bevorzugterweise ist das bei der Erfindung zum Einsatz kommende Reservoir als Ampulle oder Beutel ausgebildet. Solche Abpackungen sind als hygienische Medikamentenreservoire bestens erprobt und erlauben zudem eine einfache Förderung des in ihnen enthaltenen Medikaments, z.B. indem ein den Ampullenboden bildender Stopfen in der Ampulle verschoben wird oder indem der Beutel von aussen her unter Druck gesetzt wird. Dabei weisen Ampullen gegenüber Beuteln den Vorteil auf, dass sie aufgrund ihrer Stabilität und Form auf einfache Weise mit einer Förderleitung verbindbar sind, während Beutel hingegen den Vorteil aufweisen, dass sie relativ wenig Verpakkungsmüll generieren und für die Förderung aus diesen durch Druckbeaufschlagung praktisch keine Reibarbeit verrichtet werden muss.

Bei weiteren bevorzugten Ausführungsformen der Erfindung kommt Insulin als flüssiges Medikament zum Einsatz, da hierbei die Vorteile der Erfindung, insbesondere bei der Behandlung des Dämmerungsphänomens bei Diabetikern, besonders deutlich zu Tage treten.

Dabei ist es bevorzugt, wenn das verwendete Reservoir mit einer Insulinmenge entsprechend 5 bis 15 internationale Insulineinheiten bereitgestellt wird, bevorzugterweise mit einer Insulinmenge entsprechend 8 bis 12 internationale Insulineinheiten, bevorzugterweise entsprechend etwa 10 internationale Insulineinheiten, da dieses die typischen Medikamentendosen zur Behandlung des Dämmerungsphänomens sind.

Auch ist es je nach Anwendungsfall vorgesehen, entweder ein Normalinsulin zu verwenden und dieses innerhalb von höchstens einer Stunde zur Infusionskanüle zu fördern, oder aber ein schnell wirkendes Insulinanalogon zu verwenden und dieses innerhalb eines Zeitraumes von mehreren Stunden zur Infusionskanüle zu fördern. Dabei kann es, je nach verwendeter Fördervorrichtung, sinnvoll sein, ein hochkonzentriertes Insulin (z.B. in der handelsüblichen Konzentration U100) zu verwenden oder ein mit einem Lösungsmittel, vorzugsweise physiologische Kochsalzlösung, verdünntes Insulin, wobei sich im letztgenannten Fall der Vorteil ergibt, dass eine genaue Dosierung aufgrund des verhältnismässig grossen spezifischen Volumens der Insulindosis vereinfacht wird.

Bei einer weiteren bevorzugten Ausführungsform der Erfindung erfolgt die Förderung des flüssigen Medikamentes zur Infusionskanüle zwischen Förderbeginn und Förderende mit einer im Wesentlichen konstanten Förderate, wodurch sich zumindest bei mehrstündiger Förderdauer der Vorteil einer im wesentlichen gleichmässigen Medikamentenausschüttung über diesen Zeitraum ergibt, was insbesondere bei der Verwendung von schnell wirkenden Insulinanaloga von Vorteil ist.

Bei noch einer weiteren bevorzugten Ausführungsform der Erfindung wird bei der Aktivierung der erfindungsgemässen Anordnung, z.B. durch Betätigen eines bestimmten Aktivierungsschalters durch den gleichzeitig eine bestimmte Vorlaufzeit festgelegt wird, oder vorgängig zur Aktivierung der Anordnung, z.B. durch Einprogrammieren einer bestimmten Vorlaufzeit oder der Uhrzeit des gewünschten Förderbeginns, die Verzögerung bis zum Beginn der Förderung gewählt oder eingestellt. Hierdurch wird die Anpassung der Medikamentenförderung an das Bedarfsprofil deutlich erleichtert.

Unabhängig davon, ob das Medikamentenreservoir in Form einer Einwegabpackung des Medikaments, in Form eines wiederbefüllbaren Mehrwegbehältnisses oder in Form einer kurz vor der Verwendung mit dem flüssigen Medikament zu ergänzenden Einwegabpackung eines physiologischen Lösungsmittels bereitgestellt wird, ist es bevorzugt, wenn die Fördervorrichtung der erfindungsgemässen Anordnung mehrfach verwendet wird, während das Reservoir jeweils ausgetauscht oder wiederbefüllt wird, da sich auf diese Weise Kosten sparen lassen. Hierfür sind Reservoir und Fördervorrichtung auf einfache Weise werkzeuglos voneinander trennbar und ebenso wieder zusammenfügbar.

Kommt als Medikamentenreservoir eine Einwegabpackung des Medikaments oder eine kurz vor der Verwendung mit dem flüssigen Medikament zu ergänzende Einwegabpackung eines physiologischen Lösungsmittels zum Einsatz, so ist es gemäss einer anderen bevorzugten Ausführungsform der Erfindung vorgesehen, sowohl die Fördervorrichtung als auch das Reservoir als Einwegartikel für die einmalige Benutzung auszugestalten und zu verwenden, indem diese zusammen als zumindest werkzeuglos untrennbare Einheit ausgebildet werden. Insbesondere bei Ausführungsformen, bei denen Einwegabpackungen des flüssigen Medikamentes als Reservoir zum Einsatz kommen, ergibt sich hierdurch eine grösstmögliche Sicherheit bei gleichzeitig maximalem Anwendungskomfort. Aber auch wenn solche Einweganordnungen mit einem anfänglich lediglich mit einem physiologischen Lösungsmittel befüllten Reservoir bereitgestellt werden, welche dann kurz vor der Anwendung, beispielweise durch Injektion des Medikamentes über ein Septum in das im Reservoir befindliche Lösungsmittel, mit dem Medikament "geladen" werden, ergibt sich ein hohes Sicherheits- und Komfortniveau, bei gleichzeitig grösstmöglicher Flexibilität betreffend das Medikament und die Medikamentenmenge.

Bei bevorzugten Ausführungsformen der Erfindung erfolgt die zeitverzögerte Auslösung der Förderung des flüssigen Medikamentes mittels einer elektronischen Steuerung, wodurch eine besonders exakte Auslösung möglich wird, insbesondere auch die Auslösung zu einer bestimmten Uhrzeit.

Bei anderen bevorzugten Ausführungsformen der Erfindung erfolgt die zeitverzögerte Auslösung der Medikamentenförderung auf rein mechanischem Wege, indem die Anordnung eine rein mechanische Auslösevorrichtung, bevorzugterweise mit hydraulischer oder festkörperreibungsbehafteter Hemmung einer Auslösebewegung, aufweist. Hierdurch ergibt sich der Vorteil, dass auf elektrische Energiequellen verzichtet werden kann und einfache, kostengünstige und zwangsablaufende Zuführungsanordnungen möglich werden, welche zuverlässig in ihrer Funktion und ausreichend präzise sind.

In einer weiteren bevorzugten Ausführungsform wird die Verzögerung mittels eines mechanischen Hemmwerks mit Anker und Unruhe, ähnlich eines mechanischen Weckers, realisiert.

Bei noch einer anderen bevorzugten Ausführungsform der Erfindung erfolgt die zeitverzögerte Auslösung der Förderung des flüssigen Medikamentes durch Ablauf einer chemischen Reaktion, bevorzugterweise indem durch diese die Betätigungsenergie für eine Auslösevorrichtung der Anordnung und/oder für die Fördervorrichtung der Anordnung erzeugt wird. Geeignete chemische Reaktionen sind beispielsweise langsam anlaufende chemische Reaktionen, welche zum Schluss stark exotherm oder unter einer starken Volumen- bzw. Druckzunahme verlaufen. Zu nennen wären hier beispielsweise exotherme Oxidationsreaktionen, bei denen die Reaktionsgeschwindigkeit mit zunehmender Temperatur zunimmt oder die Reaktion zweier Grundkomponenten zu einem Kunststoffschaum, z.B. Polyurethan-Schaum, welche mit einer grossen Volumenzunahme einhergeht. Zudem ist es auch vorgesehen, durch Elekrolyse ein unter zunehmendem Druck stehendes Gas zu erzeugen, mittels dessen Druck die Medikamentenförderung ausgelöst und/oder bewerkstelligt wird.

Bei einer bevorzugten Ausführungsform der Erfindung kommt elektrische Energie als ausschliessliche oder zumindest teilweise Antriebsenergie zum Einsatz, und zwar bevorzugterweise derart, dass ein Elektromotor der Fördervorrichtung der erfindungsgemässen Anordnung mit elektrischem Strom angetrieben wird oder der Heizwiderstand eines Bimetallantriebs, eines Dehnstoffaktuators oder eines Formgedächtnisaktuators derselben mit elektrischem Strom betrieben wird. Hierzu verfügt die Anordnung über eine elektrische Energiequelle, welche bevorzugterweise von einer Einwegbatterie oder einem wiederaufladbaren Akkumulator gebildet ist. Der Vorteil solcher Ausführungsformen ist darin zu sehen, dass hierdurch eine grosse Flexibilität hinsichtlich der Steuerbarkeit des Förderungsverlaufs möglich wird.

Bei einer weiteren bevorzugten Ausführungsform der Erfindung kommt als Antriebsenergie für die Förderung des flüssigen Medikaments ausschliesslich oder zumindest teilweise mechanische Energie aus einem mechanischen Energiespeicher zum Einsatz, und zwar bevorzugterweise aus einer vorgespannten Feder oder einem komprimierten Gas. Solche Energiespeicher sind denkbar einfach und zuverlässig und können zudem bei kleiner und leichter Bauweise relativ grosse Energiemengen speichern. Entsprechend ergibt sich der Vorteil, dass hierdurch die Realisierung kostengünstiger und zugleich zuverlässiger erfindungsgemässer Anordnungen begünstigt wird.

Bei noch einer weiteren bevorzugten Ausführungsform der Erfindung kommt als Antriebsenergie für die Förderung des flüssigen Medikaments ausschliesslich oder zumindest teilweise Energie aus einer chemischen Reaktion zum Einsatz, bevorzugterweise aus einer unter Ausdehnung, Druckaufbau und/oder Wärmeentwicklung stattfindenden chemischen Reaktion. Dabei ist es für den Fall, dass auch zur Auslösung der Förderung des flüssigen Medikamentes eine chemische Reaktion verwendet wird, bevorzugt, das dieselbe chemische Reaktion sowohl zur Auslösung als auch zur Bereitstellung der Antriebsenergie für die Förderung des flüssigen Medikaments verwendet wird. Beispiele für geeignete chemische Reaktionen wurden bereits zuvor erwähnt und sind hier noch um das bevorzugte Beispiel einer elektrischen oder chemischen Zündung eines pyrotechnischen Treibsatzes zu ergänzen, mittels welchem die Antriebsenergie für die Förderung des flüssigen Medikaments bereitgestellt werden kann.

Bei noch einer weiteren bevorzugten Ausführungsform der Erfindung kommt als Antriebsenergie für die Förderung des flüssigen Medikaments ausschliesslich oder zumindest teilweise Energie aus einem osmotischen Druckaufbau zum Einsatz.

Die beiden letztgenannten Ausführungsformen weisen den Vorteil auf, dass sich zumindest bei Grossserienproduktion sehr kostengünstige und zuverlässige erfindungsgemässe Anordnungen herstellen lassen, was deren Auslegung und Verwendung als Einwegartikel wirtschaftlich interessant erscheinen lässt.

Um das fluidische "Totvolumen" der erfindungsgemässen Anordnung und damit die nicht nutzbare Menge flüssigen Medikamentes so gering wie möglich zu halten, ist es generell bevorzugt, die Verbindung zwischen Reservoir und Kanüle so kurz wie möglich zu gestalten, zum Beispiel indem diese über einen sehr kurzen Überleitungsschlauch von wenigen cm Länge miteinander verbunden sind. Noch bevorzugter ist es jedoch, die Anordnung derartig auszubilden, dass die Kanüle bei der Aktivierung der erfindungsgemässen Anordnung oder spätestens bei der Auslösung der Medikamentenförderung derselben ein Septum des Reservoirs durchsticht oder von vornherein selbst einen Reservoirbestandteil bildet. Hierdurch ergibt sich zusätzlich der Vorteil, dass aufwändige und potentiell fehleranfällige Koppelstellen vermieden werden.

Ein dritter und ein vierter Aspekt der Erfindung bestehen darin, dass das erfindungsgemässe Verfahren angewendet bzw. die erfindungsgemässe Anordnung verwendet wird zum Zuführen eines flüssigen Medikamentes, und zwar bevorzugterweise Insulin, in den Körper eines Patienten, was bevorzugterweise während der frühen Morgenstunden erfolgt. Bei dieser Anwendung bzw. Verwendung treten die Vorteile der Erfindung besonders deutlich zu Tage.

Ein fünfter Aspekt der Erfindung betrifft eine Einwegabpackung eines Insulins oder eines Insulinanalogons zur Verwendung als Reservoir für eine Anordnung gemäss dem zweiten Aspekt der Erfindung, welche eine Insulinmenge bzw. Insulinanalogonmenge entsprechend 5 bis 15, bevorzugterweise von 8 bis 12 und bevorzugterweise von 10 internationale Einheiten Insulin enthält.

Ein sechster Aspekt der Erfindung betrifft eine Einwegabpackung eines physiologischen Lösungsmittels, bevorzugterweise einer physiologischen Kochsalzlösung, zur Bildung eines Reservoirs für eine Anordnung gemäss dem zweiten Aspekt der Erfindung, mit einem Septum zum Zuführen eines flüssigen Medikamentes mittels einer Spritze oder eines sogenannten Medikamenten-Pens in das in der Einwegabpackung abgepackte Lösungsmittel. Durch Injizieren einer entsprechenden Menge Insulin kann aus dieser Einwegabpackung eine Einwegabpakkung gemäss dem fünften Aspekt der Erfindung gebildet werden.

Die Abpackungen gemäss dem fünften und den sechsten Aspekt stellen bevorzugte Handelswaren dar und kommen sowohl als Teil von erfindungsgemässen Einweganordnungen als auch als Teil von erfindungsgemässen Anordnungen, bei denen die Fördervorrichtung mehrfach verwendet wird, zum Einsatz.

Weitere bevorzugte Ausführungen der Erfindung ergeben sich aus den abhängigen Ansprüchen und aus der nun folgenden Beschreibung anhand der Figuren. Dabei zeigen:
die Figuren 1a und 1b idealisierte zeitliche Verläufe des abgegebenen Medikamentenvolumens V und der Förderrate Q einer erfindungsgemässen Anordnung im Betrieb;
die Figuren 2a bis 2c schematisch eine erste erfindungsgemässe Anordnung vor Beginn, während und nach Beendigung der Medikamentenförderung;
die Figuren 3a und 3b schematisch eine zweite erfindungsgemässe Anordnung vor und bei Beginn der Medikamentenförderung;
die Figuren 4a und 4b schematisch eine dritte erfindungsgemässe Anordnung vor und nach Beginn der Medikamentenförderung;
die Figuren 5a und 5b schematisch eine vierte erfindungsgemässe Anordnung vor Beginn und am Ende der Medikamentenförderung;
die Figuren 6a und 6b schematisch eine fünfte erfindungsgemässe Anordnung vor Beginn und nach Beendigung der Medikamentenförderung; und
die Figuren 7a und 7b schematisch eine sechste erfindungsgemässe Anordnung vor Beginn und kurz vor Beendigung der Medikamentenförderung.

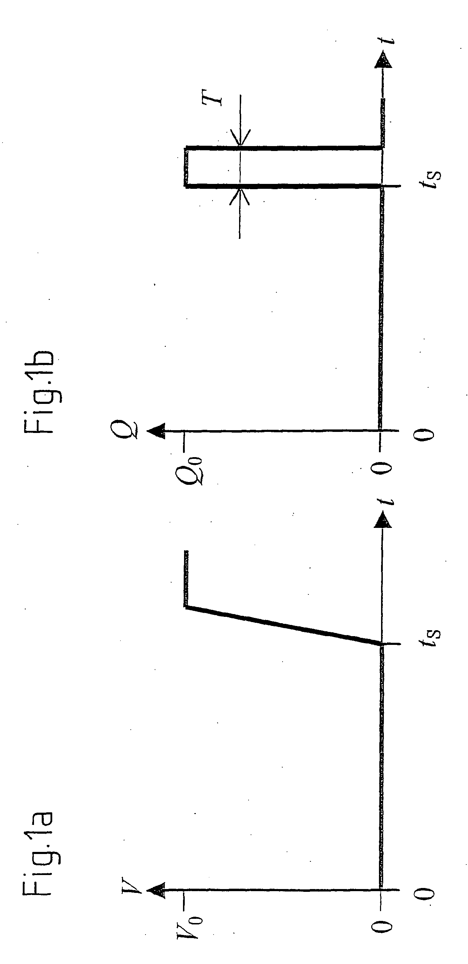

Das grundsätzliche Funktionsprinzip der Erfindung lässt sich anhand der Figuren 1a und 1b erklären, welche die idealisierten zeitlichen Verläufe des abgegebenen Medikamentenvolumens V (Fig. 1a) und der Förderrate Q (Fig. 1b) einer erfindungsgemässen Anordnung beim Zuführen von Insulin in den Körper eines Patienten zur Behandlung des sogenannten "Dämmerungsphänomens" zeigen. Wie zu erkennen ist, erfolgt nach dem Aktivieren der Anordnung (t = 0) zunächst bis zum Startzeitpunkt ts der Förderung keine oder nur eine vernachlässigbar kleine Ausschüttung des flüssigen Medikaments (V = 0, Q = 0). Beginnend mit ts wird sodann über die relativ kurze Zeit T ein Insulinvolumen V₀ gemäss Fig. 1a in den Körper des Patienten gefördert.

Die tatsächlichen Verläufe können je nach Realisierung der erfindungsgemässen Anordnung von den in den Figuren 1a und 1b gezeigten Verläufen abweichen,
wobei der grundsätzliche Ablauf jedoch im Wesentlichen immer gleich ist.

Dieser kann in die folgenden drei Abschnitte eingeteilt werden:

| | |
|---|---|
| 1. *t*≤0 | Anlegen und Aktivierung der Anordnung (zum Zeitpunkt t = 0) |
| 2. 0<*t*<*tₛ* | Verzögerung |
| 3. *tₛ ≤t≤tₛ* +*T* | Förderung (Ausschüttung) |

Sowohl der Zeitpunkt der Ausschüttung als auch die Medikamentenmenge sind individuell unterschiedlich. Für eine Medikamentenausschüttung in den frühen Morgenstunden, z.B. die in der Figuren 1a und 1b dargestellte, beträgt die Verzögerungszeit einige Stunden. Die auszuschüttende Medikamentenmenge kann z.B. im gezeigten Fall bei V₀ = 10 IU, oder mit anderen Worten, bei 10 internationalen Insulineinheiten, liegen. Die Dauer T der eigentlichen Medikamentenabgabe richtet sich vorrangig nach dem eingesetzten Medikamententyp. Im Falle von Normalinsulin mit einer Gesamtwirkdauer von einigen Stunden sollte die Abgabe recht schnell erfolgen, typischerweise mit T < 1 Stunde. Wird dagegen z.B. ein sehr schnell wirkendes Insulinanalogon eingesetzt, erfolgt die Abgabe deutlich langsamer, also z.B. etwa mit T = 3 Stunden. Insbesondere beim Einsatz von langsam wirkenden Medikamenten wie Normalinsulin sind sowohl der genaue Startzeitpunkt der Förderung als auch das Abgabeprofil recht unkritisch. Bei schnell wirkenden Medikamenten wie z.B. bestimmten Insulinanaloga sind diese Grössen kritischer.

Die im folgenden beschriebenen Figuren zeigen schematische Darstellungen von bevorzugten Ausführungsformen erfindungsgemässer Anordnungen, welche unter anderem auch zur Durchführung der zuvor beschriebenen Insulinzuführung zur Behandlung des Dämmerungsphänomens bei Diabetikern geeignet sind.

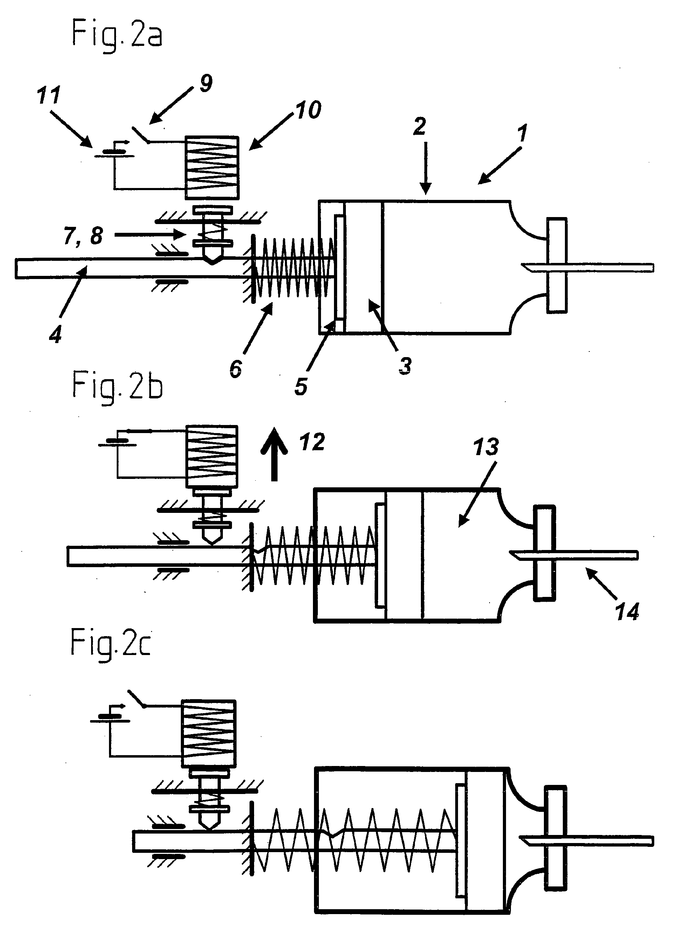

Der Grundaufbau einer ersten erfindungsgemässen Anordnung ist in den Figuren 2a bis 2c in unterschiedlichen Betriebszuständen schematisch dargestellt. Diese Anordnung weist einen Federantrieb mit elektromagnetischer Auslösung auf. Fig. 2a zeigt die Anordnung im Ruhezustand vor der Förderung, d.h. zum Zeitpunkt t < ts. Das Medikamentenreservoir 2 besteht in diesem Beispiel aus einer zylindrischen Ampulle 1 mit Stopfen 3. Der Antrieb wird von einer Kolbenstange 4 mit Flansch 5 sowie einer Druckfeder 6 gebildet. Im dargestellten Zustand vor der Insulinförderung ist die Feder 6 gespannt und die Kolbenstange 4 wird durch einen Verriegelungsmechanismus, bestehend aus einer Klinke 7 und einer Druckfeder 8, in ihrer Position arretiert. Zum Förderbeginnzeitpunkt t = ts wird durch eine (nicht dargestellte) elektronische Steuerung ein elektronischer Schalter 9 betätigt, wodurch ein Elektromagnet 10 mittels einer Energiequelle (Batterie, Akku, etc.) 11 mit elektrischem Strom beaufschlagt wird. Hierdurch wird die Sperrklinke 7 in Richtung 12 von der Kolbenstange wegbewegt und gibt diese frei. Detaillierte technische Ausführungsformen solcher Auslösevorrichtungen sind, z.B. aus der Relaistechnik, in grosser Zahl bekannt und für den Fachmann auf diese Anwendung übertragbar.

Nach Freigabe der Kolbenstange 4 entspannt sich die Antriebsfeder 6, wobei mittels Flansch 5 und Stopfen 3 das Medikament 13 aus dem Reservoir 2 verdrängt und durch die Kanüle 14 injiziert wird. Diese Situation ist in Fig. 2b dargestellt.

Der Endzustand nach vollständiger Entleerung des Reservoirs 2 ist in Fig. 2c dargestellt. Das Spannen der Antriebsfeder 6 vor der nächsten Anwendung erfolgt z.B. durch Niederdrücken des Flansches 5 mittels eines geeigneten Werkzeuges oder durch Zurückziehen der Kolbenstange 4.

Die Dauer T der Medikamentenabgabe ergibt sich im vorliegenden Fall aus der Federkraft, der Stopfenreibung des Reservoirs 2 und dem Strömungswiderstand der Kanüle 14 (zuzüglich eines eventuell vorhandenen Überleitsystems). Ohne weitere Massnahmen wird T für eine typische Auslegung im Sekundenbereich liegen. Damit ist die Anordnung in dieser Form für die Abgabe von Normalinsulin geeignet. Wird zur Applikation von Insulinanaloga eine Abgabezeit im Stundenbereich benötigt, so kann die Entspannung der Antriebsfeder 6, z.B. durch einen hydraulischen Dämpfer, gezielt verzögert werden. Besonders vorteilhaft ist es in diesem Fall jedoch, den Strömungswiderstand des Medikamentenüberleitsystems selbst zur Dämpfung heranzuziehen. Hierzu wird ein sich an das Reservoir 2 anschliessendes Überleitsystem ganz oder teilweise durch eine Glaskapillare gebildet, durch deren Länge und Innendurchmesser die Abgabezeit T in weiten Grenzen einstellbar ist.

In einer bevorzugten Ausführungsform kann es sich bei der Antriebsfeder 6 auch um eine Gasdruckfeder handeln. Das Einstellen des Abgabezeitpunktes erfolgt, je nach Ausführung der Steuerung, entweder durch Einstellung der Verzögerungszeit zwischen dem Anlegen der Anordnung und der Förderung (Ausschüttung) des Medikaments oder durch unmittelbare Einstellung der Ausschüttungsuhrzeit. Statt der elektronisch gesteuerten elektromagnetischen Auslösung sind ähnliche Systeme, wie bereits zuvor erwähnt, auch mit einem mechanischen Hemmwerk, insbesondere ein mechanisches Hemmwerk mit Anker und Unruhe, realisierbar.

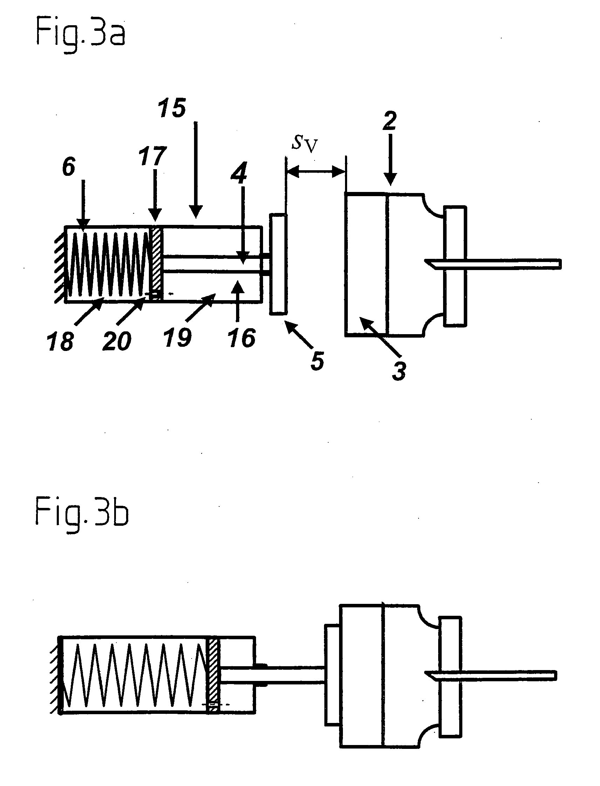

Die Figuren 3a und 3b zeigen schematisch eine zweite erfindungsgemässe Anordnung mit Federantrieb, einmal vor Förderungsbeginn (Fig. 3a) und einmal bei Förderungsbeginn (Fig. 3b). Da bei dieser Ausführungsform die Verzögerungswirkung rein mechanisch durch einen hydraulischen Dämpfer realisiert wird, benötigt sie keine elektronischen bzw. elektromechanischen Komponenten. Die Antriebsfeder 6 läuft hier in einem Dämpferzylinder 15, der mit einer hochviskosen Flüssigkeit 16 gefüllt ist. Die Antriebsfeder 6 arbeitet auf einen Dämpferkolben 17, der im Zylinder 15 spielfrei läuft und diesen in zwei Kammern 18 und 19 unterteilt. Die einzige fluidische Verbindung der beiden Kammern 18, 19 wird durch eine Bohrung (oder mehrere Bohrungen) 20 geringen Durchmessers (z.B. 0.1 mm) gebildet. Fest mit dem Dämpferkolben 17 verbunden ist eine aus dem Zylinder 15 herausgeführte Kolbenstange 4 mit Flansch 5. Die Vorspannung der Antriebsfeder 6 führt hier zur Verdrängung von Dämpferflüssigkeit 16 aus Kammer 19 in die Kammer 18 durch die Bohrung 20 hindurch. Aufgrund der hohen Viskosität der Dämpferflüssigkeit 16 sowie des geringen Bohrungsdurchmessers ergibt sich eine starke Dämpfung und eine entsprechend langsame Bewegung. Die Verzögerungswirkung ergibt sich dadurch, dass der Flansch 5 zunächst um eine gewisse Verzögerungsstrecke Sv vom Stopfen 3 des Reservoirs 2 abgerückt ist. Die Medikamentenförderung beginnt erst zu dem Zeitpunkt, zu dem der Flansch 5 den Stopfen 3 berührt. Dieser Zeitpunkt ist in Fig. 3b dargestellt. Durch geeignete Auslegung lässt sich auf diese Weise eine Verzögerungszeit von mehreren Stunden erzielen. Wird die Verzögerungsstrecke einstellbar ausgelegt, so können in Verbindung mit einer entsprechenden Einstellskala leicht unterschiedliche Verzögerungszeiten realisiert werden. Das erneute Spannen der Antriebsfeder 6 vor dem nächsten Einsatz beinhaltet eine Verdrängung der Dämpferflüssigkeit 16 von Kammer 18 zu Kammer 19 und muss mit grosser Kraft oder sehr langsam erfolgen. Besonders vorteilhaft geschieht dies durch die definierte Belastung des Flansches 5 mit einem Gewicht. Da der Einsatz der Anordnung bevorzugterweise über Nacht erfolgt, ist ein Zeitraum von mehreren Stunden zum Spannen unkritisch. Zweckmässigerweise wird eine entsprechende Vorrichtung vorgesehen, in welche der Patient die Anordnung nach dem Ablegen am Morgen einsetzt und welche das Spannen der Feder 6 während des Tages vornimmt. Es ist jedoch ebenfalls möglich, die beiden Kammern 18, 19 über eine Bypassleitung grossen Querschnitts zu verbinden, die während des Einsatzes durch ein Ventil oder ein ähnliches Element abgesperrt ist. Nach einem Öffnen des Ventils kann das Spannen der Feder 6 aufgrund des grösseren Strömungsquerschnittes nun deutlich schneller und mit geringerer Kraft erfolgen. Die hier gezeigte Anordnung ist insofern besonders vorteilhaft, als sie sich mit geringer Baugrösse realisieren lässt, es sind jedoch auch andere Anordnungen möglich. So kann z.B. auch auf eine Befüllung von Kammer 18 mit Dampferflüssigkeit sowie die Bohrung 20 verzichtet werden. Stattdessen wird dann die Kammer 19 über eine Kapillare (nicht gezeigt) mit einem Ausgleichsgefäss (nicht gezeigt) verbunden. Die Verdrängung der Dämpferflüssigkeit 16 erfolgt sodann aus Kammer 19 durch die Kapillare hindurch in das Ausgleichsgefäss. Da die Kapillare im Vergleich zur Bohrung 20 deutlich länger ist, kann ihr Durchmesser entsprechend grösser gewählt werden. Systembedingt fällt bei der in den Figuren 3a und 3b gezeigten Anordnung die Vorschubgeschwindigkeit der Kolbenstange 4 exponentiell über die Zeit ab, so dass der Förderhub deutlich kleiner als der Verzögerungshub gewählt werden sollte, damit die Ausschüttungszeit nicht zu gross wird. Das Medikamentenreservoir 2 sollte daher eine grosse Fläche bei kleinem Hub besitzen. Für einen Förderhub von etwa 1 mm wird die Ausschüttungsdauer T typischerweise im Stundenbereich liegen.

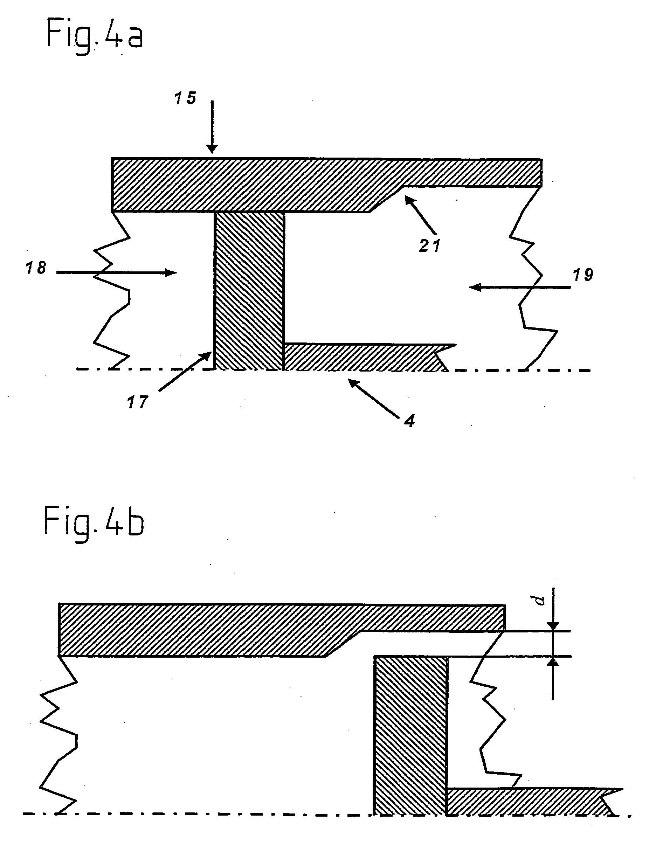

Die Figuren 4a und 4b zeigen Detailansichten eines Dämpferzylinders 15 einer dritten erfindungsgemässen Anordnung ähnlich der in den Figuren 3a und 3b gezeigten im Schnitt. Der wesentliche Unterschied gegenüber der in den Figuren 3a und 3b gezeigten Anordnung besteht jedoch darin, dass der Durchmesser des Dämpferzylinders 15 hier nicht über seine gesamte Länge konstant ist, sondern eine (nahezu) sprunghafte Änderung 21 aufweisst. In Fig. 4a ist zunächst die Situation vor der Ausschüttung, d.h. zu einem Zeitpunkt t < ts, dargestellt. Hier entsprechen die Verhältnisse der vorherigen Darstellung. Die Anfangsposition von Kolbenstange 4 und Dämpferkolben 17 im Dämpferzylinder 15 wird so gewählt, dass sich der Dämpferkolben 17 zum Zeitpunkt t = ts an der Änderungsstelle 21 des Zylinderdurchmessers befindet. Durch den für t > ts grösseren Zylinderdurchmesser entsteht zwischen Zylinder 15 und Kolben 17 ein Spalt d, durch welchen das Dämpferöl weitgehend ungehindert von Kammer 19 zu Kammer 18 strömen kann, wodurch die Dämpfung stark herabgesetzt oder gar aufgehoben wird. Entsprechend werden eine schnelle Ausschüttung und ein grösserer Förderhub, welche mit dem System gemäss den Figuren 3a und 3b nicht ohne Weiteres realisierbar sind, möglich. Zudem sind auch Ausführungsformen denkbar, bei denen eine Dämpferanordnung ähnlich der hier gezeigten nur zur Realisierung der Verzögerung eingesetzt wird, z.B. indem hiermit zum Zeitpunkt t = ts eine separate Feder als Antrieb für die Medikamentenförderung freigegeben wird.

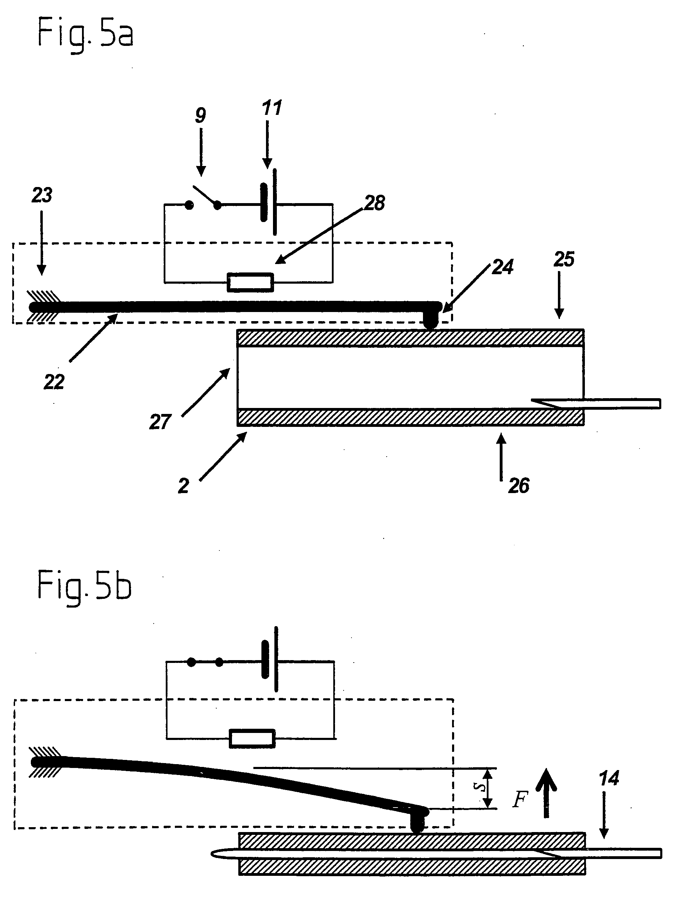

Die Figuren 5a und 5b zeigen eine vierte erfindungsgemässe Anordnung, einmal vor Förderungsbeginn (Fig. 5a) und einmal bei Ende der Förderung (Fig. 5b). Wie zu erkennen ist, weist die hier gezeigte Anordnung als Antrieb einen Bimetall-Aktuator auf (siehe gestrichelte Linie). Dieser umfasst eine Blattfeder 22 aus Thermo-Bimetall, die an ihrem einen Ende 23 fest eingespannt ist und mit ihrem anderen Ende über ein Druckstück 24 mit dem Reservoir 2 unmittelbar in Kontakt steht oder von diesem einen geringen Abstand (typischerweise < 1 mm) aufweisst. Das Reservoir 2 ist hier als Beutel mit starren Deckelflächen 25, 26 und flexiblem Seitenteil 27 ausgebildet. Das Reservoir 2 und der Bimetall-Aktuator können hier entweder gemeinsam als (teilsteriles) Einwegprodukt ausgebildet sein oder lediglich das Reservoir 2 ist als Einwegprodukt ausgebildet, während der Bimetall-Aktuator gemeinsam mit Steuerung und Energieversorgung ein wiederverwendbares Grundgerät bildet. Der Bimetall-Aktuator ist vorzugsweise so ausgelegt, dass die Bimetallfeder 22 für t < ts eine ebene Form aufweisst. Sofern das Druckstück 24 bereits in Kontakt mit dem Reservoir 2 ist, reicht die von der Bimetallfeder 22 auf das Reservoir 2 ausgeübte Kraft nicht aus, die Deckelfläche 25 des Reservoirs 2 zu verschieben. Zum Zeitpunkt t = ts bzw. kurz davor wird die Bimetallfeder 22 durch Schliessen eines elektronischen Schalters 9 mittels einer Energiequelle 11 beheizt (Fig. 5b). Die Heizung erfolgt durch einen ohm'schen Widerstand 28, welcher in engem thermischen Kontakt zur Bimetallfeder 22 steht und beispielsweise durch eine auf die Bimetallfeder 22 aufgebrachte Konstantan-Schicht gebildet sein kann. Alternativ kann der Widerstand 28 jedoch auch durch die Bimetallfeder 22 selbst gebildet werden. Weiterhin sind zur Heizung auch Elemente mit nichtlinearer Charakteristik, insbesondere Kaltleiter, einsetzbar. Durch die Erwärmung biegt sich das freie Ende der Bimetallfeder 22 in Richtung des Reservoirs 2 aus und verschiebt die Deckelfläche 25 des Reservoirs 2 um die Strecke s, wodurch das Medikament aus dem Reservoir 2 in das Überleitsystem bzw. in die Kanüle 14 verdrängt wird.

Da eine Blattfeder aus Bimetall ein relativ geringes mechanisches Arbeitsvermögen besitzt, kommen der erforderlichen Verschiebung s und der durch das Reservoir ausgeübten Gegenkraft F grosse Bedeutung zu. Daher sind Reservoirs mit grosser Grundfläche bei geringem Hub und kleiner Reibungskraft im vorliegenden Fall besonders vorteilhaft. Diese Forderungen können besonders gut durch beutelförmige Reservoirs erfüllt werden. Eine sinnvolle Auslegung ist etwa für s kleiner oder gleich 3 mm und F kleiner oder gleich 3 N möglich. Allerdings sollte die Auslegung so erfolgen, dass Biegungen der Bimetallfeder aufgrund von Schwankungen der Umgebungstemperatur nicht zu einer verfrühten Medikamentenausschüttung führen. Dies lässt sich entweder durch einen gewissen Abstand zwischen Druckstück 24 und Deckelfläche 25 für t < ts oder durch eine gewisse Reservoirreibung erreichen. Anstelle einer Bimetall-Blattfeder ist auch der Einsatz einer Bimetall-Schnappscheibe möglich, die ihre Biegungsrichtung bei Erwärmung in einem kleinen Temperaturbereich nahezu sprunghaft ändert. Für die hier nicht dargestellte elektronische Steuerung gelten alle bereits zuvor bei den Ausführungsformen mit Federantrieb gemachten Aussagen sinngemäss. Neben der Steuerung und den gezeigten Hauptkomponenten kann die Anordnung weitere Sicherheits- und Hilfseinrichtungen enthalten. Zu nennen sind hier insbesondere ein Überhitzungsschutz und eine Vorrichtung in Form eines Kontaktes, Schalters etc. zur Detektion der vollständigen Entleerung des Reservoirs, was grundsätzlich für alle erfindungsgemässen Anordnungen bevorzugt ist. Auch besteht, ebenso wie bei den nachfolgend beschriebenen weiteren Ausführungsformen mit thermischen Aktuatoren, die Möglichkeit, die eigentliche Verzögerung durch ein mechanisches Hemmwerk mit Federantrieb zu realisieren, welches nach Ablauf der Verzögerungszeit die Heizung mittels eines elektromechanischen Schalters aktiviert.

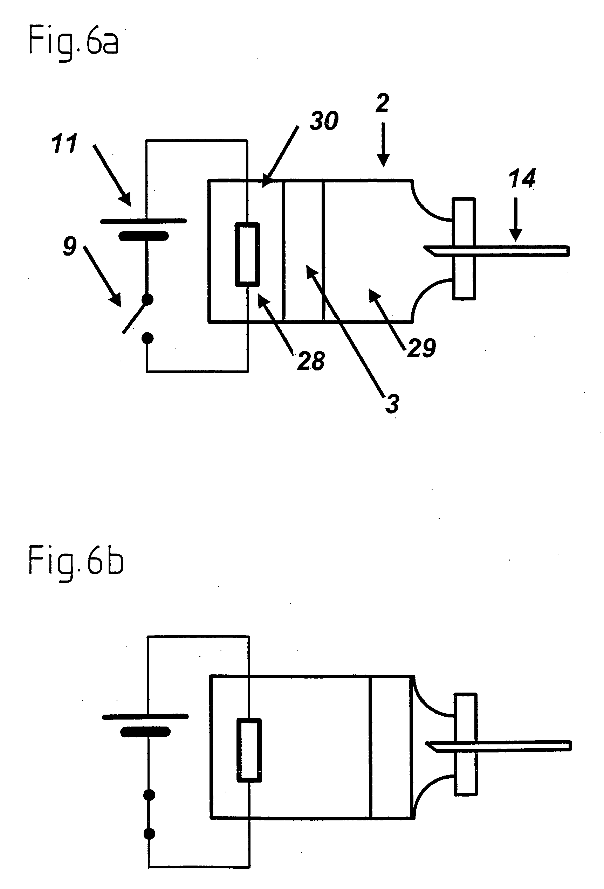

Die Figuren 6a und 6b zeigen eine fünfte erfindungsgemässe Anordnung, einmal vor Förderungsbeginn (Fig. 6a) und einmal nach Beendigung der Förderung (Fig. 6b). Wie zu erkennen ist, weist die hier gezeigte Anordnung als Antrieb für die Förderung des flüssigen Medikamentes einen Dehnstoffaktuator mit elektrischer Auslösung auf. Dabei ist das Medikamentenreservoir 2, im hier gezeigten Beispiel eine zylindrische Ampulle, in zwei Kammern 29 und 30 unterteilt, die durch einen axial verschiebbaren Stopfen 3 voneinander getrennt sind. In der ersten Kammer 29 befindet sich das Medikament, während die zweite Kammer 30 mit einem sogenannten Dehnstoff gefüllt ist. Unter Dehnstoffen werden Materialien (in der Regel Wachse oder Ähnliches) verstanden, die einen relativ geringen Schmelzpunkt besitzen und beim Schmelzen eine grosse Volumenzunahme erfahren. In Fig. 6a ist zunächst die Situation t < ts dargestellt, bei der der Dehnstoff sich im festen Zustand befindet. Zum Zeitpunkt t = ts bzw. kurz davor wird ein vom Dehnstoff umgebener Heizwiderstand 28 gemäss Fig. 6b durch Schliessen eines elektronischen Schalters 9 mit einer elektrischen Energiequelle 11 verbunden. Hierdurch wird der Dehnstoff aufgeheizt und schmilzt. Aufgrund der hiermit verbundenen Volumenzunahme wird der Stopfen 3 in axialer Richtung verschoben und verdrängt das Medikament aus der ersten Kammer 29 in das Überleitsystem bzw. die Kanüle 14. Anstelle eines festen Dehnstoffes können auch Flüssigkeiten wie Alkohohle mit geringer spezifischer Wärmekapazität und grosser thermischer Ausdehnung eingesetzt werden. Während sich bei der hier gezeigten Ausführung Dehnstoff und Medikament in einem gemeinsamen Reservoir befinden und der Dehnstoffaktuator ein Einwegartikel ist, sind selbstverständlich auch Ausführungen mit einem wiederverwendbaren Aktuator vorgesehen. Ebenso kann der Heizwiderstand 28 wahlweise auch ausserhalb der Dehnstoffkammer 30 angeordnet sein. Zudem ist es auch möglich, die zweite Kammer 30 statt mit einem Dehnstoff mit einer Elektrolytlösung vollständig zu füllen und die Verdrängung des Stopfens 3 durch eine elektrolytische Gaserzeugung vorzunehmen.

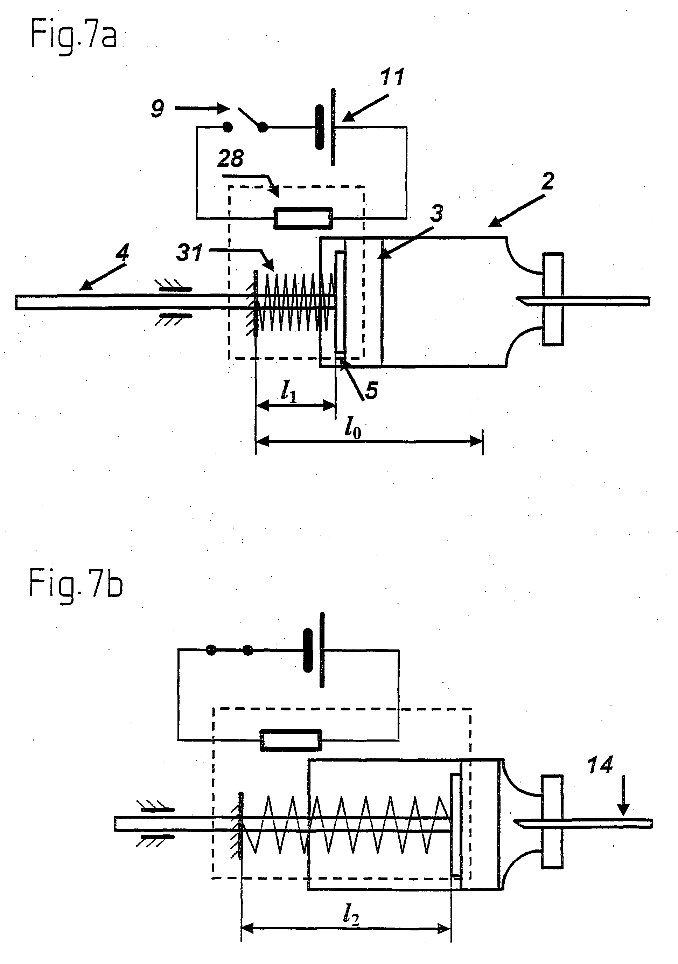

Die Figuren 7a und 7b zeigen eine sechste erfindungsgemässe Anordnung, einmal vor Förderungsbeginn (Fig. 7a) und einmal kurz vor Ende der Medikamentenförderung (Fig. 7b). Wie zu erkennen ist, weist die hier gezeigte Anordnung als Antrieb für die Förderung des flüssigen Medikamentes einen Formgedächtnis-Aktuator mit elektrischer Auslösung auf (siehe gestrichelte Linie). Der Formgedächtnis-Aktuator umfasst ein Formgedächtniselement 31 (z.B. auf Nickel-Titan-Basis), welches hier die Form einer Spiralfeder der Ruhelänge l₀ besitzt, eine Kolbenstange 4 und einen Flansch 5. In Fig. 7a ist die Situation vor Beginn der Ausschüttung, d.h. zu einem Zeitpunkt t < ts, dargestellt. Hier ist das Formgedächtniselement 31 auf die im Vergleich zu l₀ geringere Länge li zusammengedrückt und berührt den Stopfen 3 des gefüllten Medikamentenreservoirs 2. Zum Zeitpunkt t = ts bzw. kurz davor wird der Formgedächtnis-Aktuator durch Schliessen einen elektronischen Schalters 9 mittels einer Energiequelle 11 beheizt (Fig. 7b). Die Heizung erfolgt entweder durch einen ohm'schen Widerstand 28, welcher in engem thermischen Kontakt zum Aktuator steht, oder der ohm'sche Widerstand des Formgedächtniselements 31 wird unmittelbar zur Heizung genutzt. Durch die Erhitzung ändert die das Formgedächtniselement bildende Aktuatorfeder 31 in einem eng begrenzten Temperaturbereich ihr kristallographisches Gefüge vom martensitischen zum austenitischen Zustand und versucht hierbei, ihre "eingeprägte" Ruhelänge l₀ einzunehmen. Bei der Ausdehnung der Aktuatorfeder 31 wird der Reservoirstopfen 3 verschoben und das Medikament aus dem Reservoir 2 in das Überleitsystem bzw. die Kanüle 14 verdrängt. Die Auslegung erfolgt vorzugsweise so, dass die sich bei vollständig geleertem Reservoir 2 ergebende Federlänge l₂ etwas geringer ist als 1₀. Auf diese Weise ist sichergestellt, dass zum Ausdrücken des Reservoirs 2 genügend Kraft zur Verfügung steht. Das Zurückstellen vor der nächsten Benutzung kann (nach Abkühlung des Formgedächtniselements 31) durch Drücken auf den Flansch 5 oder ein Zurückziehen der Kolbenstange 4 erfolgen. Da hier im Gegensatz zu den zuvor beschriebenen Federantrieben nur eine recht geringe Kraft erforderlich ist, kann das Drücken des Flansches 5 ggf. auch automatisch beim Einsetzen des neuen Reservoirs 2 durch dessen Stopfen 3 erfolgen. Voraussetzung hierfür ist eine über der Rückstellkraft liegende Haftreibungskraft des Reservoirstopfens 3 oder die Verwendung eines zunächst ausgangsseitig verschlossenen Reservoirs. Weiterhin kann die Rückstellung automatisch durch eine parallel zum Formgedächtniselement 31 angeordnete Zugfeder erfolgen. Im vorliegenden Beispiel ist der Formgedächtnis-Aktuator Teil eines widerverwendbaren Grundgerätes. Es ist jedoch auch vorgesehen, ihn als Teil des Einweg-Reservoirs auszuführen.

Anstelle der zuvor gezeigten, elektrischen Widerstandsheizungen zur Bereitstellung von Wärme für die Bimetall-, Dehnstoff- und Fomgedächtnisaktuatoren ist es auch vorgesehen, chemische Wärmequelle als Heizung einzusetzen. Diese können entweder sofort bei Aktivierung der Anordnung aktiviert werden, um sodann eine relativ langsame exotherme Reaktion in Gang zu setzen, oder aber verzögert durch eine mechanische oder elektronische Steuerung, um dann relativ schnell Reaktionswärme zu erzeugen.

Neben den zuvor dargelegten Fördermechanismen lässt sich eine verzögerte Medikamentenausschüttung auch durch Verwendung anderer Antriebsenergiequellen realisieren. So ist es beispielsweise auch vorgesehen, die Medikamentenförderung dadurch zu bewirken, dass zum Zeitpunkt ts ein zuvor komprimiertes Antriebsgas (beispielsweise Kohlendioxid) entspannt wird und durch seine hierbei auftretende Ausdehnung das Medikament aus seinem Reservoir verdrängt. Als Gasreservoir wird bevorzugterweise eine vorgefüllte Gaspatrone eingesetzt, wobei die verzögerte Auslösung bevorzugterweise mittels eines elektronisch oder mechanisch gesteuerten Hemmwerks erfolgt. Des Weiteren ist es vorgesehen, die Medikamentenförderung durch die vorzugsweise elektrische Zündung eines kleinen pyrotechnischen Treibsatzes zu bewirken, dessen Explosion mit einer grossen Expansion verbunden ist. Der Treibsatz selbst und die Zündelektroden bilden dabei einen Teil des Medikamentenreservoirs und sind Einwegartikel, während die elektronische Steuerung sowie die Vorrichtung zur Erzeugung der Zündspannung zu einem wiederverwendbaren Grundgerät gehören. Anstatt eines Feststofftreibsatzes kann auch eine entsprechend kleine Menge eines explosiven Gases eingesetzt werden.

Als weitere Antriebsenergiequelle sind auch osmotische Antriebe vorgesehen, bei denen die gerichtete Diffusion eines Lösungsmittels durch eine semipermeable Membran zur Erzeugung einer Druckkraft eingesetzt wird. Dabei kann der osmotische Antrieb bei den erfindungsgemässen Anordnungen auf zwei verschiedene Weisen eingesetzt werden. Entweder ist er als osmotische Pumpe aufgebaut, welche durch den osmotischen Druck das flüssige Medikament aus dem Reservoir verdrängt, wobei die Verzögerung der Förderung durch einen Leerstellweg erzielt wird, ähnlich wie dies bei der in den Figuren 3a und 3b gezeigten Anordnung der Fall ist. Oder der sich nach der Aktivierung (Beginn der osmotischen Diffusion) zunächst langsam aufbauende osmotische Druck wird lediglich zur Verzögerung benutzt, der mittels einer geeigneten Vorrichtung bei Überschreitung eines Schwellwertes die Medikamentenausschüttung initiiert, z.B. indem ein Federantrieb freigegeben wird, welcher dann die für die Förderung erforderliche Energie bereitstellt.

## Patentansprüche

1. Verfahren zur automatisierten Zuführung eines flüssigen Medikamentes (13) zu einer Infusionskanüle (14), umfassend die Schritte:
a) Bereitstellen eines Reservoirs (1, 2) mit einer bei kurzfristiger subkutaner Verabreichung für einen Menschen nicht-tödlichen Menge des flüssigen Medikamentes (13), und
b) Fördern des flüssigen Medikamentes (13) mittels einer automatisierten Fördervorrichtung vom Reservoir (1, 2) zu der Infusionskanüle (14),
wobei die Fördervorrichtung insbesondere manuell aktiviert wird und sodann mit einer Verzögerung zwischen vier und acht Stunden automatisch mit der Förderung des flüssigen Medikamentes (13) beginnt und wobei nach Beginn der Förderung die gesamte im Reservoir (1, 2) bereitgestellte Menge des flüssigen Medikamentes (13) innerhalb eines Zeitraumes von maximal vier Stunden zur Infusionskanüle (14) gefördert wird.

2. Verfahren nach Anspruch 1, **dadurch gekennzeichnet, dass** das Reservoir (1, 2) in Form einer Einwegabpackung des flüssigen Medikamentes (13) oder in Form eines wiederbefüllbaren Behältnisses enthaltend das flüssige Medikament (13) bereitgestellt wird.

3. Verfahren nach einem der vorangehenden Ansprüche, **dadurch gekennzeichnet, dass** das Reservoir (1, 2) **dadurch** bereitgestellt wird, dass in eine Einwegabpackung eines physiologischen Lösungsmittels für flüssige Medikamente (13), insbesondere in eine Einwegabpackung physiologischer Kochsalzlösung, insbesondere über ein Septum eine bestimmte Menge des flüssigen Medikamentes (13) eingebracht wird.

4. Verfahren nach einem der vorangehenden Ansprüche, **dadurch gekennzeichnet, dass** das flüssige Medikament (13) mit einem als Ampulle (1) oder Beutel ausgebildeten Reservoir (1, 2) bereitgestellt wird.

5. Verfahren nach einem der vorangehenden Ansprüche, **dadurch gekennzeichnet, dass** als flüssiges Medikament (13) Insulin verwendet wird.

6. Verfahren nach Anspruch 5, **dadurch gekennzeichnet, dass** ein Reservoir (1, 2) mit einer Insulinmenge entsprechend 5 bis 15 internationale Insulineinheiten bereitgestellt wird, insbesondere mit einer Insulinmenge entsprechend 8 bis 12 internationale Insulineinheiten, insbesondere entsprechend 10 internationale Insulineinheiten.

7. Verfahren nach einem der Ansprüche 5 bis 6, **dadurch gekennzeichnet, dass** ein Normalinsulin verwendet wird und die gesamte bereitgestellte Insulinmenge innerhalb eines Zeitraumes (T) von maximal einer Stunde zur Infusionskanüle (14) gefördert wird.

8. Verfahren nach einem der Ansprüche 5 bis 6, **dadurch gekennzeichnet, dass** ein schnell wirkendes Insulinanalogon verwendet wird und die gesamte bereitgestellte Insulinmenge innerhalb eines Zeitraumes (T) von zwei bis vier Stunden zur Infusionskanüle (14) gefördert wird.

9. Verfahren nach einem der vorangehenden Ansprüche, **dadurch gekennzeichnet, dass** das flüssige Medikament (13) zwischen Beginn und Ende der Förderung mit einer im Wesentlichen konstanten Förderate (Q₀) zur Infusionskanüle (14) gefördert wird.

10. Verfahren nach einem der vorangehenden Ansprüche, **dadurch gekennzeichnet, dass** bei der Aktivierung oder vorgängig zu der Aktivierung der Fördervorrichtung die Verzögerung bis zum Beginn der Förderung gewählt oder eingestellt wird.

11. Verfahren nach einem der vorangehenden Ansprüche, **dadurch gekennzeichnet, dass** die Fördervorrichtung mehrfach verwendet wird während das Reservoir (1, 2) jeweils ausgetauscht wird.

12. Verfahren nach einem der Ansprüche 1 bis 10, **dadurch gekennzeichnet, dass** die Fördervorrichtung und das Reservoir (1, 2) mehrfach verwendet werden, wobei das Reservoir (1, 2) neu befüllt wird.

13. Verfahren nach einem der Ansprüche 1 bis 10, **dadurch gekennzeichnet, dass** die Fördervorrichtung und das Reservoir (1, 2) nur einmal benutzt werden.

14. Verfahren nach einem der vorangehenden Ansprüche, **dadurch gekennzeichnet, dass** die Förderung des flüssigen Medikamentes (13) mittels einer elektronischen Steuerung ausgelöst wird.

15. Verfahren nach einem der Ansprüche 1 bis 13, **dadurch gekennzeichnet, dass** die Förderung des flüssigen Medikamentes (13) mittels einer rein mechanischen Auslösevorrichtung, insbesondere mit hydraulischer Hemmung, ausgelöst wird.

16. Verfahren nach einem der Ansprüche 1 bis 13, **dadurch gekennzeichnet, dass** die Förderung des flüssigen Medikamentes (13) durch Ablauf einer chemischen Reaktion ausgelöst wird.

17. Verfahren nach einem der vorangehenden Ansprüche, **dadurch gekennzeichnet, dass** die Antriebsenergie für die Förderung des flüssigen Medikamentes (13) zumindest teilweise aus einer elektrischen Energiequelle (11) bereitgestellt wird, insbesondere aus einer Batterie (11), und insbesondere, dass mit der Energiequelle (11) ein Motor angetrieben wird oder der Heizwiderstand (28) eines Bimetallantriebs, eines Dehnstoffaktuators oder eines Formgedächtnisaktuators betrieben wird.

18. Verfahren nach einem der vorangehenden Ansprüche, **dadurch gekennzeichnet, dass** die Antriebsenergie für die Förderung des flüssigen Medikamentes (13) zumindest teilweise aus einem mechanischen Energiespeicher (6, 31) bereitgestellt wird, insbesondere aus einer vorgespannten Feder (6) oder einem komprimierten Gas.

19. Verfahren nach einem der vorangehenden Ansprüche, **dadurch gekennzeichnet, dass** die Antriebsenergie für die Förderung des flüssigen Medikamentes (13) zumindest teilweise aus einer chemischen Reaktion bereitgestellt wird, insbesondere aus einer unter Ausdehnung, Druckaufbau und/oder Wärmeentwicklung stattfindenden chemischen Reaktion, insbesondere aus einer pyrotechnischen Reaktion.

20. Verfahren nach einem der vorangehenden Ansprüche, **dadurch gekennzeichnet, dass** die Antriebsenergie für die Förderung des flüssigen Medikamentes (13) durch osmotischen Druckaufbau bereitgestellt wird.

21. Verfahren nach Anspruch 16 und nach Anspruch 19, **dadurch gekennzeichnet, dass** die chemische Reaktion, welche zur Auslösung der Förderung des flüssigen Medikamentes (13) verwendet wird, zusätzlich verwendet wird zur Bereitstellung der Antriebsenergie für die Förderung des flüssigen Medikamentes (13).

22. Anwendung des Verfahrens nach einem der vorangehenden Ansprüche zum Zuführen eines flüssigen Medikamentes (13), insbesondere von Insulin, in den Körper eines Patienten, insbesondere während der frühen Morgenstunden.

23. Anordnung zum automatisierten Zuführen eines flüssigen Medikamentes (13) in den Körper eines Patienten, insbesondere zur Durchführung des Verfahrens nach einem der Ansprüche 1 bis 21, umfassend eine Infusionskanüle (14) zur subkutanen Einbringung des Medikamentes (13) in den Körper, ein Reservoir (1, 2) enthaltend eine bei kurzfristiger subkutaner Verabreichung für einen Menschen nicht-tödliche Menge eines flüssigen Medikamentes (13) und/oder eines physiologischen Lösungsmittels für ein flüssiges Medikament (13) sowie eine automatisierte Fördervorrichtung zur Förderung des flüssigen Medikamentes (13) von dem Reservoir (1, 2) zur Infusionskanüle (14), wobei die Fördervorrichtung derartig ausgebildet ist, dass sie nach einer insbesondere manuellen Aktivierung mit einer Verzögerung von vier bis acht Stunden automatisch mit der Förderung des flüssigen Medikamentes (13) beginnt und nach Beginn der Förderung die gesamte im Reservoir (1, 2) bereitgestellte Fluidmenge (V₀) innerhalb eines Zeitraumes (T) von maximal vier Stunden zur Infusionskanüle (14) fördert.

24. Anordnung nach Anspruch 23, **dadurch gekennzeichnet, dass** das Reservoir (1, 2) eine Einwegabpackung eines flüssigen Medikamentes (13) ist oder ein wiederbefüllbares Behältnis, welches ein flüssiges Medikament (13) enthält.

25. Anordnung nach Anspruch 23, **dadurch gekennzeichnet, dass** das Reservoir (1, 2) eine Einwegabpackung eines physiologischen Lösungsmittels für ein flüssiges Medikament (13), insbesondere eine Einwegabpackung physiologischer Kochsalzlösung ist.

26. Anordnung nach Anspruch 25, **dadurch gekennzeichnet, dass** das Reservoir (1, 2) ein Septum zum Injizieren eines flüssigen Medikamentes (13) in seinen Innenraum aufweist.

27. Anordnung nach einem der Ansprüche 23 bis 26, **dadurch gekennzeichnet, dass** das Reservoir (1, 2) als Ampulle (1) oder Beutel ausgebildet ist.

28. Anordnung nach einem der Ansprüche 23 bis 27, **dadurch gekennzeichnet, dass** das Reservoir (1, 2) Insulin als flüssiges Medikament (13) enthält, insbesondere eine Insulinmenge entsprechend 5 bis 15 internationale Insulineinheiten, insbesondere entsprechend 8 bis 12 internationale Insulineinheiten, und insbesondere entsprechend 10 internationale Insulineinheiten.

29. Anordnung nach Anspruch 28, **dadurch gekennzeichnet, dass** das Insulin ein Normalinsulin ist und die Fördervorrichtung ausgebildet ist zur Förderung der gesamten bereitgestellten Insulinmenge (V₀) innerhalb eines Zeitraumes (T) von maximal einer Stunde.

30. Anordnung nach Anspruch 28, **dadurch gekennzeichnet, dass** das Insulin ein schnell wirkendes Insulinanalogon ist und die Fördervorrichtung ausgebildet ist zur Förderung der gesamten bereitgestellten Insulinmenge (V₀) innerhalb eines Zeitraumes (T) von zwei bis vier Stunden.

31. Anordnung nach einem der Ansprüche 23 bis 30, **dadurch gekennzeichnet, dass** die Fördervorrichtung ausgestaltet ist zur Förderung des flüssigen Medikamentes (13) mit einer zwischen Beginn und Ende der Förderung im wesentlichen konstanten Förderrate (Q₀).

32. Anordnung nach einem der Ansprüche 23 bis 31, **dadurch gekennzeichnet, dass** die Fördervorrichtung Mittel zum Wählen oder Einstellen einer bestimmten Verzögerungszeit aufweist.

33. Anordnung nach einem der Ansprüche 23 bis 32, **dadurch gekennzeichnet, dass** die Fördervorrichtung und das Reservoir (1, 2) werkzeuglos voneinander trennbar ausgebildet sind zur Ermöglichung einer mehrfachen Verwendung der Fördervorrichtung mit jeweils einem neuen oder neu befüllten Reservoir (1, 2).

34. Anordnung nach einem der Ansprüche 23 bis 32, **dadurch gekennzeichnet, dass** die Fördervorrichtung und das Reservoir (1, 2) werkzeuglos untrennbar miteinander verbunden sind.

35. Anordnung nach einem der Ansprüche 23 bis 34, **dadurch gekennzeichnet, dass** die Fördervorrichtung eine elektronische Steuerung aufweist zur Auslösung der Förderung des flüssigen Medikamentes (13).

36. Anordnung nach einem der Ansprüche 23 bis 34, **dadurch gekennzeichnet, dass** die Fördervorrichtung eine rein mechanische Auslösevorrichtung, insbesondere mit hydraulischer Hemmung, aufweist, zur Auslösung der Förderung des flüssigen Medikamentes (13).

37. Anordnung nach einem der Ansprüche 23 bis 36, **dadurch gekennzeichnet, dass** die Fördervorrichtung eine Auslösevorrichtung aufweist, welche eine Auslösung der Förderung des flüssigen Medikamentes (13) infolge Ablaufs einer chemischen Reaktion bewirkt.

38. Anordnung nach einem der Ansprüche 23 bis 37, **dadurch gekennzeichnet, dass** die Anordnung eine elektrische Energiequelle (11) zur zumindest teilweisen Bereitstellung der Antriebsenergie für die Förderung des flüssigen Medikamentes (13) aufweist, insbesondere eine Batterie, und insbesondere, dass die Anordnung einen mit der Energiequelle (11) antreibbaren Motor aufweist oder einen Bimetallantrieb, einen Dehnstoffaktuator oder einen Formgedächtnisaktuator aufweist, dessen Heizwiderstand (28) mit der Energiequelle (11) betreibbar ist.

39. Anordnung nach einem der Ansprüche 23 bis 38, **dadurch gekennzeichnet, dass** die Anordnung einen mechanischen Energiespeicher (6, 31), insbesondere eine vorgespannte Feder (6) oder ein mit komprimiertem Gas gefülltes Behältnis aufweist, zur zumindest teilweisen Bereitstellung der Antriebsenergie für die Förderung des flüssigen Medikamentes (13).

40. Anordnung nach einem der Ansprüche 23 bis 39, **dadurch gekennzeichnet, dass** die Anordnung derartig ausgestaltet ist, dass die Antriebsenergie für die Förderung des flüssigen Medikamentes (13) zumindest teilweise aus einer chemischen Reaktion bereitgestellt wird, insbesondere aus einer unter Ausdehnung, Druckaufbau und/oder Wärmeentwicklung stattfindenden chemischen Reaktion, und insbesondere, dass die Anordnung einen pyrotechnischen Treibsatz zur Bereitstellung der Antriebsenergie für die Förderung des flüssigen Medikamentes (13) aufweist.

41. Anordnung nach einem der Ansprüche 23 bis 40, **dadurch gekennzeichnet, dass** die Anordnung einen osmotischen Antrieb zur Bereitstellung der Antriebsenergie für die Förderung des flüssigen Medikamentes (13) aufweist.

42. Anordnung nach Anspruch 37 und nach Anspruch 40, **dadurch gekennzeichnet, dass** die Anordnung eine Reaktionskammer aufweist, in welcher bei Aktivierung der Anordnung eine chemische Reaktion in Gang gesetzt wird, welche nach einer Verzögerungszeit die Antriebs-energie für die Förderung des flüssigen Medikamentes (13) liefert.

43. Verwendung der Anordnung nach einem der Ansprüche 23 bis 42 zum automatisierten Zuführen eines flüssigen Medikamentes (13), insbesondere von Insulin, in den Körper eines Patienten, insbesondere während der frühen Morgenstunden.

44. Einwegabpackung Insulin zur Verwendung als Reservoir (1, 2) für eine Anordnung nach einem der Ansprüche 23 bis 42, enthaltend 5 bis 15, insbesondere 8 bis 12 und insbesondere 10 internationale Einheiten Insulin.

45. Einwegabpackung eines physiologischen Lösungsmittels, insbesondere einer physiologischen Kochsalzlösung, zur Bildung eines Reservoirs (1, 2) für eine Anordnung nach einem der Ansprüche 23 bis 42, mit einem Septum zum Zuführen eines flüssigen Medikamentes (13) mittels einer Spritze in das in der Einwegabpackung abgepackte Lösungsmittel.
